# EUROPÄISCHE PATENTSCHRIFT

(11) **EP 2 051 704 B1**
(45) Veröffentlichungstag und Bekanntmachung des Hinweises auf die Patenterteilung: **18.04.2012**
(21) Anmeldenummer: 06793706.0
(22) Anmeldetag: 21.09.2006
(51) Int. Cl.: A61K 9/28, A61K 9/00, A61K 9/50, A61K 31/00

(54) **Pharmazeutische Zusammensetzung mit kontrollierter Wirkstoffabgabe für Wirkstoffe mit guter Löslichkeit in Wasser**
Pharmaceutical composition with controlled active ingredient delivery for active ingredients with good solubility in water
Composition pharmaceutique à libération contrôlée de substance active pour substances actives présentant une bonne solubilité dans l'eau

(30) Priorität: 18.08.2006 IN CH14652006
(43) Veröffentlichungstag der Anmeldung: 29.04.2009
(73) Patentinhaber: Evonik Röhm GmbH, 64293 Darmstadt (DE)
(72) Erfinder: PETEREIT, Hans-Ulrich, 64291 Darmstadt (DE); RAVISHANKAR, Hema, Mumbay 400089 (IN); BODINGE, Shradda, Mumbai 400002 (IN)
(86) Internationale Anmeldenummer: PCT/EP2006/066583
(87) Internationale Veröffentlichungsnummer: WO 2008/019712

(56) Entgegenhaltungen:
- EP-A1- 0 436 370
- EP-A1- 1 157 690
- EP-A1- 1 213 015
- DE-A1- 19 845 358
- DE-A1-102004 035 938
- US-A1- 5 395 628

## Beschreibung

### Gebiet der Erfindung

Die Erfindung betrifft das Gebiet der pharmazeutischen Zusammensetzungen mit kontrollierter Wirkstoffabgabe für Wirkstoffe mit guter Löslichkeit in Wasser.

### Stand der Technik

EP-A 0 463 877 beschreibt pharmazeutische Zusammensetzungen mit verzögerter Wirkstofffreisetzung, bestehend aus einem Kern mit einem pharmazeutischen Wirkstoff und einem einschichtigen Überzugsfilm der ein wasserabweisendes Salz und ein wasserunlösliches Copolymer aus Ethylacrylat, Methylmethacrylat und Trimethylammoniumethlymethacrylat-Chlorid enthält. Das wasserabweisende Salz kann z. B. Ca- oder Mg-Stearat sein. Es werden sigmoide Freisetzungskurven erhalten.

EP-A 0 225 085, EP-A 0 122 077 und EP-A 0 123 470 beschreiben die Verwendung organischer Säure in Arzneimittelkernen, die mit verschiedenen Überzügen aus organischen Lösungen versehen werden. Es resultieren im Wesentlichen sigmoide Freisetzungscharakteristiken. EP-A 0 436 370 beschreibt pharmazeutische Zusammensetzungen mit verzögerter Wirkstofffreisetzung, bestehend aus einem Kern mit einem pharmazeutischen Wirkstoff und einer organischen Säure und einem äußeren Überzugsfilm der durch wässriges Sprühen aufgebracht wurde und ein Copolymer aus Ethylacrylat, Methylmethacrylat und Trimethylammoniumethlymethacrylat-Chlorid ist. Dabei werden ebenfalls sigmoide Freisetzungskurven erhalten.

EP 1 117 387 B1 beschreibt ein ähnliches System wie EP-A 0 436 370.
Der wesentliche Unterschied besteht in der Verwendung der organischen Säuren in der Salzform, wodurch insbesondere die Dauer der Lag-Phase als auch die Steilheit der sigmoiden Freigabekurven günstig beeinflusst werden können. Als mögliche Verarbeitungshilfsmittel werden unter anderem gemahlene Kieselsäure und Porenbildner genannt.

### Aufgabe und Lösung

Bei einer Vielzahl von Arzneiformen bzw. Wirkstoffen ist eine sigmoide Freisetzungscharakteristik mit einer Anfangsphase mit verzögerter Wirkstofffreisetzung (Lag-Phase), einer sich anschließenden Haupt-Freisetzungsphase (Pulse-Phase) und einer Auslaufphase therapeutisch sinnvoll. Generell soll in der Lag-Phase eine möglichst niedrige Wirkstofffreisetzung und in der sich anschließenden Pulse-Phase eine möglichst rasche Wirkstofffreisetzung stattfinden. Dieses Ziel wird teilweise durch die technischen Lehren der EP-A 0 436 370 und EP 1 117 387 B1 erreicht. In der EP 1 117 387 B1 werden vor allem mit Natrium-Acetat gute Resultate erzielt. Es besteht jedoch ein ständiger Verbesserungsbedarf. Es wurde als eine Aufgabe gesehen, die aus EP-A 0 436 370 und EP 1 117 387 B1 bekannten pharmazeutischen Präparationen mit sigmoider Wirkstofffreisetzung weiterzuentwickeln, um den oben genannten Zielen näher zu kommen.

Die Aufgabe wurde gelöst durch eine Pharmazeutische Zubereitung, enthaltend
a) einen Kern mit einem Wirkstoff und einer organischen Säure und/oder dem Salz einer organischen Säure
b) einen den Kern umschließenden Überzug, der einen Polymeranteil aus (Meth)acrylatcopolymeren enthält, die nicht mehr als 15 Gew.-% an kationischen oder anionischen Gruppen aufweisen und der zu mindestens 60 Gew.-% eines (Meth)acrylat-Copolymeren enthält, das sich aus radikalisch polymerisierten Einheiten von 93 bis 98 Gew.-% C₁- bis C₄-Alkylestern der Acryl- oder der Methacrylsäure und 7 bis 2 Gew.-% (Meth)acrylat-Monomeren mit einer quaternären Ammoniumgruppe im Alkylrest zusammensetzt,
   dadurch gekennzeichnet, dass
   der Wirkstoff eine Wasserlöslichkeit von mindestens 10 g/l bei 20 °C aufweist und
   der Überzug Siliciumdioxid-Teilchen mit einer mittleren Teilchengröße im Bereich von 1 bis 50 µm enthält.

### Ausführung der Erfindung

### Kerne (a)

Im einfachsten Fall kann der Kern nur aus dem Wirkstoff und der organischen Säure und/oder dem Salz der organischen Säure zusammengesetzt sein, in der Regel enthält er zusätzlich einen Träger, z. B. ein Nonpareill, und pharmazeutische Hilfsstoffe, wie z. B. hochdisperse Kieselsäure oder Polyvinylpyrrolidon (PVP).

Der Kern (a) kann beispielsweise bestehen aus:
- Wirkstoff in einer Menge von 97,5 bis 2,5, bevorzugt 80 bis 5 Gew.%, bezogen auf das Kerngewicht
- einer organischen Säure und/oder einem oder mehreren Salzen von organischen Säuren in einer Menge von 2,5 bis 97,5, bevorzugt 5 bis 80, insbesondere 10 bis 50 Gew.%, bezogen auf das Kerngewicht
- optional pharmazeutischen Hilfsstoffen in einer Menge von 0 - 95, bevorzugt 10 bis 50 Gew.%, bezogen auf das Kerngewicht
- optional einem Träger mit einem Anteil am Kerngewicht von 0 bis 95, bevorzugt 10 bis 50 Gew.-%

Die Kerne können z. B. durch direktes Verpressen, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln hergestellt werden.

Die zusätzlich zum Wirkstoff enthaltenen pharmazeutischen Hilfsstoffe können z. B. Bindemittel, wie Cellulose und deren Derivate, Polyvinylpyrrolidon (PVP), Gelatine, (Meth)acrylate, Stärke und deren Derivate oder Zucker sein.

Die Kerne können homogen sein oder einen schichtartigen Aufbau aufweisen, wobei sich der Wirkstoff bevorzugt in der äußeren Schicht befindet. Besonders bevorzugt bildet die organische Säure und/oder das Salz der organischen Säure die äußere Schicht des Kerns.

### Organische Säuren

Die eingesetzten organischen Säuren müssen toxikologisch unbedenklich und in Arzneimitteln verwendbar sein. Der bevorzugte Typ hängt von der speziellen Formulierung ab. Bevorzugt sind organische Säuren wie Citronensäure, Fumarsäure, Ameisensäure, Bernsteinsäure, Essigsäure, Maleinsäure, Weinsäure, Glutarsäure oder Milchsäure.

Besonders geeignet für die Zwecke der Erfindung ist Bernsteinsäure. Zitronensäure ist im Prinzip ebenfalls geeignet, allerdings werden in gepufferten Medien, die in etwa physiologischen Verhältnissen entsprechen, keine so steilen Freigabeprofile wie mit Succinat erhalten. Essigsäure kann gelegentlich zu Stabilitätsproblemen führen, die im Zuge der Lagerung der Arzneiformen auftreten können. Derartige Probleme sind bei Einsatz von Bernsteinsäure bisher nicht bekannt.

Der Typ der Säure kontrolliert die Steilheit der Wirkstofffreigabekurve insbesondere bei sigmoiden Freigabekurven.

In den erfindungsgemäßen Formulierungen können die organischen Säuren bevorzugt als äußere Schicht des Kerns vorliegen, gebunden durch Bindemittel. Sie können durch Aufsprühen aus Lösung oder durch pulverförmigen Auftrag unter gleichzeitiger Zugabe von Bindemittellösung aufgebracht werden.

In Einzelfällen sind jedoch auch Varianten sinnvoll, in denen der Wirkstoff in Mischung mit organischen Säuren aufgetragen wird oder zwischen der Wirkstoffschicht und der Salzschicht eine Isolierschicht aufgetragen wird. Die organische Säure kann auch zuletzt auf den Kern aufgetragen werden, so dass sie die äußere Schicht bildet.

Der Anteil der organischen Säure(n) am Kerngewicht kann 2,5 Gew.-% bis 97,5 Gew.-%, bevorzugt 5 bis 80 Gew.-%, insbesondere 10-50 Gew.-% betragen.

### Salze organischer Säuren:

Bevorzugt gegenüber den organischen Säuren sind die Salze organischer Säuren. In den meisten Fällen wird im Vergleich zu den organischen Säuren beim Einsatz der organischer Säuren in der Salzform eine geringere Wirkstofffreigabe während der Lag-Phase und anschließend eine raschere Wirkstofffreisetzung beobachtet.

Die eingesetzten Salze organischer Säuren müssen toxikologisch unbedenklich und in Arzneimitteln verwendbar sein. Bevorzugt sind Alkalisalze (Ammonium, Lithium, Natrium, Kalium). Der bevorzugte Typ hängt von der speziellen Formulierung ab; neben der erfindungsgemäßen Funktionalität sind jedoch auch die pharmakologischen Effekte der Ionen zu berücksichtigen. Bevorzugt sind Salze schwacher organischer Säuren wie Bernsteinsäure, Citronensäure, Fumarsäure, Ameisensäure, Essigsäure, Maleinsäure, Weinsäure, Glutarsäure oder Milchsäure. Besonders geeignet für die Zwecke der Erfindung ist Natrium-Succinat. NatriumCitrat ist im Prinzip ebenfalls geeignet, allerdings werden in gepufferten Medien, die in etwa physiologischen Verhältnissen entsprechen, keine so steilen Freigabeprofile wie mit Natrium-Succinat erhalten. Natrium-Acetat kann gelegentlich zu Stabilitätsproblemen führen, die im Zuge der Lagerung der Arzneiformen auftreten können. Derartige Probleme sind bei Einsatz von Natrium-Succinat bisher nicht bekannt.

Der Typ der Säure kontrolliert die Steilheit Wirkstoffgabekurve insbesondere bei sigmoiden Freigabekurven.

In den erfindungsgemäßen Formulierungen können die Salze als äußere Schicht des Kerns vorliegen, gebunden durch Bindemittel. Sie können durch Aufsprühen aus Lösung oder durch pulverförmigen Auftrag unter gleichzeitiger Zugabe von Bindemittellösung aufgebracht werden.

In Einzelfällen sind jedoch auch Varianten sinnvoll, in denen der Wirkstoff in Mischung mit den Salzen aufgetragen wird oder zwischen der Wirkstoffschicht und der Salzschicht eine Isolierschicht aufgetragen wird. Das Salz der organischen Säure kann auch zuletzt auf den Kern aufgetragen werden, so dass es die äußere Schicht bildet.

Der Anteil der Salze der organischen Säure(n) am Kerngewicht kann 2,5 Gew.-% bis 97,5 Gew.-%, bevorzugt 5 bis 80 Gew.-%, insbesondere 10 - 50 Gew.-% betragen.

### Überzug b)

Der Überzug b), besteht aus einem oder mehreren (Meth)acrylat-Copolymeren, SiO₂-Teilchen, sowie gegebenenfalls aus üblichen pharmazeutischen Hilfsstoffen, wie z. B. Weichmachern, Pigmenten, Netzmitteln, Trennmitteln etc.. Der äußere Überzug umschließt den Kern bevorzugt unmittelbar, ohne dass sich zwischen dem Kern und dem Überzugsfilm weitere Schichten befinden.

Der Polymeranteil des Überzugs ist zusammen mit den enthaltenen SiO₂-Teilchen und gegebenenfalls enthaltenen weiteren Hilfsstoffen wie z. B. Weichmachern verfilmt und bildet einen durchgängigen Überzug bzw. Überzugsfilm. Der Überzug bzw. Überzugsfilm in seiner Gesamtheit steuert zusammen mit der im Kern vorhandenen organischen Säure und/oder deren Salzen die Wirkstoffabgabe.

### Polymeranteil des Überzugs b)

Der Polymeranteil des Überzugs b) enthält zumindest 60 Gew.-%, bevorzugt zu 85 bis 95 Gew.-% ein oder gegebenenfalls auch mehrere (Meth)acrylat-Copolymere, aus radikalisch polymerisierten Monomer-Einheiten, bestehend zu 93 bis 98 Gew.-% aus C₁- bis C₄-Alkylestern der Acryl- oder der Methacrylsäure und zu 7 bis 2 Gew.-% aus (Meth)acrylat Monomeren mit einer quaternären Ammoniumgruppe im Alkylrest (Typ EUDRAGIT® RS). Gegebenenfalls kann der Polymeranteil des Überzugs auch zu 100 % aus dem vorstehenden Polymertyp bestehen.

Der Polymeranteil des Überzugs kann bezogen auf das Gewicht des Kerns bevorzugt 10 bis 200, bevorzugt 20 bis 100 Gew.-% betragen.

Der Polymeranteil des Überzugs bezogen auf den Überzug soll mindestens 50 Gew.-% betragen. Der Überzug kann gegebenenfalls nur aus den genannten (Meth)acrylatcopolymeren und dem SiO₂-Anteil bestehen. In der Regel wird der Überzug jedoch zusätzlich zum SiO₂-Anteil weitere pharmazeutisch übliche Zusatzstoffe, wie z. B. Weichmacher oder Pigmente, enthalten.

Das Erfindungsprinzip beruht auf einer vermuteten Wechselwirkung zwischen den essentiellen Bestandteilen des Kerns und den essentiellen Bestandteilen des Überzugs.

Überraschenderweise tritt die erfindungsgemäße Wirkung nur bei Wirkstoffen ein, die eine Wasserlöslichkeit von mindestens 10 g/l, bevorzugt mindestens 30g/l mehr bevorzugt mindestens 100 g/l aufweisen. Um diese Wechselwirkung sicherzustellen, soll das genannte (Meth)acrylat-Copolymere zu mindestens 50 Gew.-% am Aufbau des Überzugs beteiligt sein, um die gewünschte Wechselwirkung zu erreichen. Derartige (Meth)acrylat-Copolymere sind handelsüblich und werden seit langem für retardierende Überzüge verwendet. Sie sind praktisch wasserunlöslich. Sie können allein oder Mischung mit anderen (Meth)acrylat-Copolymeren verwendet werden.

Um erfindungsgemäß sigmoide Wirkstofffreisetzungscharakteristiken zu realisieren, soll der Polymeranteil des Überzugs b) zu mindestens 60, bevorzugt zu mindestens 85 Gew.-% oder zu 100 Gew.-% aus dem genannten Copolymer-Typ (Eudragit® RS-Typ) bestehen.

Bevorzugte C₁- bis C₄-Alkylestern der Acryl- oder der Methacrylsäure sind Methylacrylat, Ethylacrylat, Butylacrylat, Butylmethacrylat und Methylmethacrylat.

Als (Meth)acrylat Monomer mit einer quaternären Ammoniumgruppe wird 2-Trimethylammoniumethylmethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-% - Trimethylammoniumethylmethacrylat-Chlorid aufgebaut sein.

Ein bevorzugtes Copolymer enthält 65 Gew.-% Methylmethacrylat, 30 Gew.-% Ethylacrylat und 5 Gew.-% 2-Trimethylammoniumethylmethacrylat-Chlorid aufgebaut (EUDRAGIT® RS)

### Mischungen von (Meth)acrylat-Copolymeren

Der Polymeranteil des Überzugs kann auch als Mischung von (Meth)acrylatcopolymeren vorliegen. Die zusätzlich für die Mischung verwendeten (Meth)acrylatcopolymeren sollen nicht mehr als 15 Gew.-% an kationischen oder anionischen Gruppen aufweisen. Bei einem Gehalt von mehr als 15 Gew.-% kationischen oder anionischen Gruppen, d.h. basischen Gruppen oder Säuregruppen im Alkylrest werden die Wechselwirkungen der Komponenten untereinander in unerwünschter bzw. kaum vorhersehbarer Weise beeinflusst.

In Falle einer Mischung beträgt der Anteil des (Meth)acrylat-Copolymeren aus radikalisch polymerisierten Monomer-Einheiten, bestehend zu 93 bis 98 Gew.-% aus C₁- bis C₄-Alkylestern der Acryl- oder der Methacrylsäure und 7 bis 2 Gew.-% (Meth)acrylat Monomeren mit einer quaternären Ammoniumgruppe im Alkylrest (Typ EUDRAGIT® RS) mindestens 60, bevorzugt 85 bis 95 Gew.-%, jeweils bezogen auf das Kerngewicht. Der Anteil des oder der zugemischten Polymere beträgt bis zu 40 Gew.-%, bevorzugt zu 5 - 15 Gew.-%, wobei sich die Anteile der gemischten Polymere zu 100 Gew.-% addieren.

Ein geeignetes (Meth)acrylat-Copolymer für eine Mischung kann z. B. aus radikalisch polymerisierten Monomer-Einheiten aus 85 bis weniger als 93 Gew.-% C₁- bis C₄-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylat-Copolymere sind handelsüblich und werden seit langem für retardierende Überzüge verwendet (Typ EUDRAGIT® RL). Der Anteil in der Mischung kann bis 40 Gew.-%, bevorzugt 5 bis 15 Gew.-% betragen.

Ein konkret geeignetes Copolymer für eine Mischung enthält z. B. 60 Gew.-% Methylmethacrylat, 30 Gew.-% Ethylacrylat und 10 Gew.-% 2-Trimethylammoniumethylmethacrylat-Chlorid (EUDRAGIT® RL).

Ein weiteres geeignetes (Meth)acrylat-Copolymer für eine Mischung besteht zu 95 bis 100, insbesondere mehr als 95 bis 100 Gew.-% aus C₁- bis C₄-Alkylestern der Acryl- oder der Methacrylsäure und bis zu 5 Gew.-% bzw. zu 0 bis 5, insbesondere 0 bis weniger als 5 Gew.-% aus Acryl- oder Methacrylsäure. Derartige (Meth)acrylatCopolymere sind handelsüblich (Typ EUDRAGIT® NE).

Der (Meth)acrylat-Copolymer-Anteil des äußeren Überzugsfilms b) kann z. B. eine Mischung sein aus:

60 bis 99, bevorzugt 85 bis 95 Gew.-% eines (Meth)acrylat-Copolymeren, das zu 93 bis 98 Gew.-% aus C₁- bis C₄-Alkylestern der Acryl- oder der Methacrylsäure und 2 bis 7 Gew.-% (Meth)acrylat-Monomeren mit einer quaternären Ammoniumgruppe im Alkylrest besteht, und

1 - 40, bevorzugt 5 bis 15 Gew.-% eines (Meth)acrylat-Copolymeren, das zu 85 bis weniger als 93 Gew.-% aus C₁- bis C₄-Alkylestern der Acryl- oder der Methacrylsäure und mehr als 7 bis 15 Gew.-% (Meth)acrylat-Monomeren mit einer quaternären Ammoniumgruppe im Alkylrest aufgebaut ist.

Bevorzugte C₁- bis C₄-Alkylestern der Acryl- oder der Methacrylsäure sind Methylacrylat, Ethylacrylat, Butylacrylat, Butylmethacrylat und Methylmethacrylat.

Als (Meth)acrylat Monomer mit einer quaternären Ammoniumgruppe wird 2-Trimethylammoniumethlymethacrylat-Chlorid besonders bevorzugt.

### Herstellung der (Meth)acrylat-Copolymere allgemein

Die (Meth)acrylat-Copolymere sind in an sich bekannter Weise durch radikalische Substanz-, Lösungs-, Perl- oder Emulsionspolymerisation erhältlich. Sie können z. B. als extrudiertes Granulat, gemahlenes Pulver, als Lösung oder Dispersion vorliegen.

### Überzüge

Der Polymerauftrag hängt von der Größe und Oberfläche der Kerne, von der Löslichkeit der Wirkstoffe und von dem angestrebten Freigabeprofil ab. Der Polymeranteil des Überzugs bezogen auf das Gewicht des Kerns kann 10 bis 200, bevorzugt 15 bis 100 Gew.-% betragen.

Die Überzüge können mehrschichtig oder als Mischung aufgetragen werden. Mischungen der Polymere erlauben die Einstellung bestimmter Steigungen in der zweiten Phase des Freigabeprofils. Der Gehalt an quaternären Ammoniumgruppen im Überzug regelt die Permeabilität und somit die Diffusionsgeschwindigkeit gelöster Substanzen (McGinity, Ed., Aqueous Polymeric Coatings for Pharmaceutical Dosage Forms, Marcel Dekker, Inc., Chapter 4, S .208-216). Je höher der Anteil an hydrophilen, quaternären Ammoniumgruppen, desto schneller ist die Freigabegeschwindigkeit. Man erhält so eine zusätzliche Steuermöglichkeit für die Wirkstoffabgabe in der zweiten Phase des Freigabeprofils.

### Zusätzliche äußere Schichten

Die erfindungsgemäße Zubereitung kann zusätzlich mit einem (Meth)acrylat-Copolymer, welches 5-60 Gew.-% Methacrylsäure-Reste enthält, umhüllt sein. Auf diese Weise kann die Zubereitung mit einer magensaftresistenten jedoch darmsaftlöslichen Umhüllung versehen werden.

Weiterhin geeignet sind anionische (Meth)acrylat-Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

EUDRAGIT® L ist ein Copolymer aus 50 Gew.-% Methylmethacrylat und 50 Gew.-% Methacrylsäure.

EUDRAGIT® L100-55 ist ein Copolymer aus 50 Gew.-% Ethylacrylat und 50 Gew.-% Methacrylsäure. EUDRAGIT® L 30D-55 ist eine Dispersion enthaltend 30 Gew.-% EUDRAGIT® L 100-55.

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S).

Besonders gut geeignet sind (Meth)acrylat Copolymere, bestehend aus 10 bis 30 Gew.-%, Methylmethacrylat, 50 bis 70 Gew.-% Methylacrylat und 5 bis 15, bevorzugt 8 bis 12 Gew.-% Methacrylsäure (Typ EUDRAGIT® FS).

EUDRAGIT® FS ist ein Copolymer aus 25 Gew.-%, Methylmethacrylat, 65 Gew.-% Methylacrylat und 10 Gew.-% Methacrylsäure. EUDRAGIT® FS 30 D ist eine Dispersion enthaltend 30 Gew.-% EUDRAGIT® FS.

Weiterhin geeignet für die Zwecke der Erfindung ist ein Copolymer (s. WO 2003/072087) welches sich aus
20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
zusammensetzt, mit der Maßgabe, dass die Glastemperatur des Copolymers nach ISO 11357-2, Punkt 3.3.3, höchstens 60 °C beträgt. Dieses (Meth)acrylatcopolymer ist wegen seiner guten Reißdehungseigenschaften insbesondere zum Verpressen von Pellets zu Tabletten geeignet.

Das Copolymer setzt sich insbesondere zusammen aus radikalisch polymerisierten Einheiten von
20 bis 34, bevorzugt 25 bis 33, besonders bevorzugt 28 bis 32 Gew.-% Methacrylsäure oder Acrylsäure, bevorzugt ist Methacrylsäure,
20 bis 69, bevorzugt 35 bis 65, besonders bevorzugt 35 bis 55 Gew.-% Methylacrylat und gegebenenfalls
0 bis 40, bevorzugt 5 bis 35, besonders bevorzugt 15 bis 35 Gew.-% Ethylacrylat zusammen, mit der Maßgabe, dass die Glastemperatur des Copolymers (Messung ohne Weichmacherzusatz bei einem Restmonomergehalt (REMO) von weniger als 100 ppm, Aufheizrate 10 °C/min, Stickstoffatmosphare) nach ISO 11357-2, Punkt 3.3.3 (*T*_{mg}), höchstens 60, bevorzugt 40 bis 60, besonders bevorzugt 45 bis 55 °C beträgt.

Das Copolymer besteht bevorzugt in wesentlichen bis ausschließlich aus den Monomeren Methacrylsäure, Methylacrylat und Ethylacrylat in den oben angegebenen Mengenanteilen.

Es können jedoch zusätzlich, ohne dass dies zu einer Beeinträchtigung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

Zur Einstellung spezieller Freisetzungsprofile bzw. Freisetzungsorte können auch Mischungen der genannten Copolymere zum Einsatz kommen.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Die Copolymere werden in an sich bekannter Weise durch radikalische Substanz-; Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden.
Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die z. B. mit ca. 6 bar Druck betrieben wird.

Weiterhin geeignet für die Zwecke der Erfindung sind Copolymere (s. WO 2004/096185) aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren, wobei sich die Anteile der Monomeren zu 100 Gew.-% addieren,
mit der Maßgabe, dass die Glastemperatur des Copolymers (glass transition temperature) nach ISO 11357-2, Punkt 3.3.3 (midpoint temperature *T*_{mg}), 55 bis 70 °C beträgt. Copolymere dieses Typs sind wegen ihrer guten mechanischen Eigenschaften insbesondere zum Verpressen von Pellets zu Tabletten geeignet.

Das oben genannte Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 25 Gew.-% Methylacrylat,
20 bis 40, bevorzugt 25 bis 35, besonders bevorzugt 28 bis 32 Gew.-% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Zur Einstellung spezieller Freisetzungsprofile bzw. Freisetzungsorte können auch Mischungen der genannten Copolymere zum Einsatz kommen.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew.-%, aus den Monomeren Methacrylsäure, Methylacrylat, Ethylacrylat und Butylmethacrylat in den oben angegebenen Mengenbereichen.

Es können jedoch zusätzlich, ohne dass dies zu einer Beeinträchtigung der wesentlichen Eigenschaften führen muss, geringe Mengen im Bereich von 0 bis 10, z. B. 1 bis 5 Gew.-% weiterer vinylisch copolymerisierbarer Monomere, wie z. B. Methylmethacrylat, Butylacrylat, Hydroxyethylmethacrylat, Vinylpyrrolidon, Vinylmalonsäure, Styrol, Vinylalkohol, Vinylacetat und/oder deren Derivate enthalten sein.

### Siliciumdioxid-Teilchen

Der den Kern umschließende Überzug enthält Siliciumdioxid-Teilchen mit einer mittleren Teilchengröße im Bereich von 1 bis 50 µm. Die im Überzug vorhandenen Siliciumdioxid-Teilchen übernehmen auch die Funktion als Antihaftmittel. Bei anspruchsgemäßer Verwendung dieser Art von Teilchen im Überzug stellt sich die erfindungsgemäße Wirkung, insbesondere eine Verkürzung der Pulse-Phase auf weniger als 4 Stunden, ein. Bei Verwendung von SiO₂-Teilchen anderer mittlerer Teilchengröße oder bei alleiniger Verwendung anderer Trennmittel, wie z. B. Talkum oder Glycerolmonostearat (GMS) scheinen die vorteilhaften Effekte der Erfindung überraschenderweise nicht einzutreten (s. die Beispiele).

Der Überzug enhält Siliciumdioxid-Teilchen (SiO₂-Teilchen) mit einer mittleren Teilchengröße d50, messbar z. B. mittels Laserdiffraction nach ISO 13320-1, im Bereich von 1 bis 50, bevorzugt von 1 bis 30, besonders bevorzugt 1 bis 10 µm. Die besten Resultate werden mit gefälltem und gemahlenem, z. B. nach Sol-Gel-Verfahren hergestelltem, SiO₂ erzielt. Dieser Siliciumdioxid-Typ wird gemäß Deutschem Arzneibuch, DAB 1999, auch als *Silicii dioxidium praecipitatum* bezeichnet. Bevorzugt sind natürlich Produkte bzw. Siliciumdioxid-Teilchen mit ausgewiesener pharmazeutischer Qualität bzw. in pharmazeutischer Qualität, die den Anforderungen gemäß DAB 1999 in Bezug auf Reinheit entsprechen.

Nicht geeignet für die Zwecke der Erfindung ist hochdisperses SiO₂ vom Aerosil®-Typ, das mittels Flamm-Verfahren hergestellt wird und meist mittlere Teilchengrößen im Bereich unter 100 nm aufweist. Letzteres kann jedoch in unkritischer Weise z. B. als Hilfsstoff bei der Formulierung der Kerne eingesetzt werden.

Bevorzugte Einsatzmengen für die Siliciumdioxid-Teilchen sind 5 bis 50, besonders bevorzugt 10 bis 40 und insbesondere 10 bis 30 Gew.-% SiO₂ bezogen auf das Trockengewicht des oder der (Meth)acrylatcopolymere im Überzug.

### Weitere pharmazeutisch übliche Hilfsstoffe

Der Kern und/oder der Überzug können weitere pharmazeutisch übliche Hilfsstoffe enthalten.

Weitere Zusatzstoffe dienen vor allem als Verarbeitungshilfsmittel und sollen ein sicheres und reproduzierbares Herstellungsverfahren sowie gute Langzeitlagerstabilität gewährleisten. Sie können die Permeabilität der Überzüge beeinflussen, was ggf. als zusätzlicher Steuerparameter genutzt werden kann:

Die Kerne können z. B. durch direktes Verpressen, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln hergestellt werden. Die zusätzlich zum Wirkstoff enthaltenen pharmazeutischen Hilfsstoffe können z. B. Bindemittel, wie Cellulose und deren Derivate, Polyvinylpyrrolidon (PVP), Gelatine, (Meth)acrylate, Stärke und deren Derivate oder Zucker sein.

### - Weichmacher:

Weichmacher können insbesondere im Überzug bzw. in den (Meth)acrylatcopolymeren des Überzugs enthalten sein. Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Ammoniumgruppen. Häufig handelt es sich um Ester, die bei Raumtemperatur flüssig sind: Citrate, Phthalate, Sebacate oder Rizinusöl. Beispiele geeigneter Weichmacher sind Citronensäurealkylester, z.,B. Triethylcitrat, Glycerinester, Phthalsäurealkylester, Sebacinsäurealkylester, Sucroseester, Sorbitanester, Diethylsebacat, Dibutylsebacat und Polyethylenglykole 4000 bis 20.000. Bevorzugte Weichmacher sind Triethylcitrat und Acetyltriethylcitrat.
Weichmacher können z. B in Mengen von 5 bis 25 Gew.-% bezogen auf das oder gegebenenfalls die Polymere des Überzugs enthalten sein.

### - Antihaftmittel:

Die im Überzug vorhandenen Siliciumdioxid-Teilchen übernehmen auch die Funktion als Antihaftmittel. In der Regel und bevorzugterweise sind daher keine weiteren Antihaftmittel notwendig und enthalten. Es ist jedoch nicht ausgeschlossen, dass zusätzlich weitere Antihaftmittel verwendet werden können.

Diese Substanzen, die in der Regel lipophile Eigenschaften besitzen, können den den Sprühsuspensionen zugesetzt werden und verhindern, zusätzlich zum erfindungsgemäß enthaltenen SiO₂, ein Agglomeration der Kerne während der Befilmung. Es können z. B. Talkum oder nicht ionische Emulgatoren, wie z. B. Glycerolmonostearat, mit einem HLB-Wert zwischen 3 und 8 eingesetzt werden. Die Mengen können zwischen 1 und 100 Gew % bezogen auf das Polymere liegen. Es ist jedoch stets darauf zu achten, dass keine Beeinträchtigung des erfindungsgemäß angestrebten Freigabeprofils auftritt.

### - Weitere Hilfsstoffe

Als weitere pharmazeutisch übliche Hilfsstoffe können in an sich bekannter Weise z. B. Stabilisatoren, Farbstoffe, Antioxidantien, Netzmittel, Porenbildner, Pigmente, Glanzmittel etc. zugegeben werden.

### Auftrag des Filmüberzuges:

Auftragsverfahren erfolgt mittels Sprühauftrag aus organischer Lösung, oder wäßrigen Dispersionen durch Schmelzen oder durch direkten Pulverauftrag. Für die Ausführung ist dabei entscheidend, dass gleichmäßige, porenfreie Überzüge entstehen.

Auftragsverfahren gemäß Stand der Technik s. z.B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation sind relevante Eigenschaften, geforderte Tests und Spezifikationen in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Wirkstoffe (Biologisch aktive Substanzen):

Die Erfindung eignet sich für Wirkstoffe, die eine Wasserlöslichkeit von mindestens 10 g/l, bevorzugt mindestens 30g/l, besonders bevorzugt von mindestens 50 g/l, insbesondere bevorzugt von mindestens 100 g/l, 200 g/l, 300 g/l oder 400 g/l bei 20 °C aufweisen (Wasserlöslichkeit in Anlehnung an Standardmethoden, wie z. B. Pharmeuropa - Technical Guide for the Elaboration of Monographs, 3rd Edition (1999), Chapter IV, Appendix IV, unter heftigem Schütteln für 1 min, 15 min stehen lassen bei 20 °C in gereinigtem Wasser). Bei Wirkstoffen mit geringerer Wasserlöslichkeit, wie z. B. Theophyllin, scheinen die vorteilhaften Wirkungen der Erfindung bei ansonsten anspruchsgemäßer Formulierung überraschender Weise nicht einzutreten.

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindem, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.
Erfindungsgemäß können alle Wirkstoffe eingesetzt werden, die die gewünschte therapeutische Wirkung im Sinne der obigen Definition erfüllen und eine ausreichende thermische Stabilität besitzen.

Die pharmazeutische Zubereitung kann beispielsweise einen oder mehrere der folgenden Wirkstoffe mit einer Wasserlöslichkeit von mindestens 10 g/l bei 20 °C gegebenenfalls in der Form der wasserlöslichen, pharmazeutisch eingesetzten Salze enthalten:

Acebutolol, Amitryptylin, Aripiprazol, Atenolol, Atropin, Betaxolol, Bisoprolol, Bupavacaine, Buproprion, Butabarbital, Carteolol, Carvedilol, Cefazoline, Cefotaxime, Chlorphenaramine, Chlorpromazine, Clindamycin, Codein, Diltiazem, Dimercaprol, Diphenhydarmine, Dopamine, Doxylamine, Duloxetine, Flexainid, Fluoxetin, Fluphenazine, Flurazepam, Gentamycin, Hydralazine, Hydrocortisone, Hydroquinone, Hyoscyamine, Isoniazid, Isoproterenol, Kanamycin, Labetolol, Lisinopril, Metipranolol, Mexiletin, Morphine, Nadolol, Neomycin, Norepinephrine, Nortryptylin, Ondansetron, Oxprenolol, Oxymetazoline, Oxymorphone, Paroxetin, Penbutolol, Phenylephrin, Pindolol, Prednisolone, Primaquine, Propranolol, Pyrrocaine, Sotalol, Sulphadiazine, Tamoxifen, Terbutalin, Timolol, Tramadol, Trazodon, Triflupromazine, Tetracycline, Tubocurarin, Venlafaxin und/oder Verapamil. Bevorzugt sind insbesondere diese Wirkstoffe in der Form der wasserlöslichen pharmazeutisch eingesetzten Salze.

Besonders bevorzugte Wirkstoffe für die Zwecke der Erfindung sind: Phenylephrin Hydrochlorid und Terbutalin Sulfat,

### Applikationsformen und weitere Ausgestaltungen

Grundsätzlich können die beschriebenen Arzneiformen direkt durch orale Applikation zur Anwendung kommen. Bevorzugt bei multipartikulären Formen (Multi Unit Dosage Form) schließt man jedoch weitere Verarbeitungsschritte an:

Erfindungsgemäß hergestellte, überzogene Arzneiformen können als Einzeldosen in Gelatinekapseln und Beutel (Sachets) oder in geeignete Mehrdosenbehälter mit Dosiereinrichtung abgefüllt werden. Die Einnahme erfolgt in fester Form oder suspendiert in Flüssigkeiten.

Durch Verpressen erhält man aus Granulaten, ggf. nach Zumischung weiterer Hilfsstoffe, Tabletten die nach der Einnahme zerfallen und die retardierten Untereinheiten freisetzen. Ebenso möglich ist die Einbettung von Agglomeraten in Polyethlenglykol oder Lipide zur Herstellung von Suppositorien oder vaginalen Arzneiformen.

Die äußeren Überzüge können zusätzlich noch mit weiteren Überzügen des Standes der Technik kombiniert bzw. überzogen werden. In diesem Sonderfall ist der äußere Überzug b) nicht der äußerste Überzug. Geeignet sind hierfür insbesondere (Meth)acrylat-Copolymere, die zu 10 bis 60 Gew.-% Methacrylsäure-Reste enthalten und im Übrigen z. B. aus Methylmethacrylat und/oder Ethylacrylat aufgebaut sind (Typ EUDRAGIT® L oder S). Auf diese Weise können in Kombination mit den erfindungsgemäßen Formulierungen zusätzlich geschmacksisolierende Eigenschaften oder Formulierungen zur gezielten Freisetzungen im Colon realisiert werden.

### Verwendung

Die erfindungsgemäße pharmazeutische Zubereitung bzw. Zusammensetzung kann zur Herstellung einer pharmazeutischen Zubereitung bzw. Zusammensetzung bzw. einer Arzneiform für Wirkstoffe, die eine Wasserlöslichkeit von mindestens 10 g/l bei 20 °C aufweisen verwendet werden, wobei die Arzneiform eine sigmoide Wirkstofffreisetzungscharakteristik mit einer Lag-Phase, einer Pulse-Phase und einer Auslaufphase aufweist, gekennzeichnet durch eine Wirkstofffreisetzung, im Paddlegerät mit 100 Upm in Puffer pH 6,8 nach Europäischem Arzneibuch, während der Lag-Phase annähernd 10 % und einer anschließenden Wirkstofffreisetzung in der Pulse-Phase von annähernd weiteren 80 % innerhalb von weniger als 4 Stunden.

Wirkstofffreisetzung nach USP siehe insbesondere USP 28-NF23, General Chapter <711>, *Dissolution,* Apparatus 2 (Paddle), Method <724> "Delayed Release (Enteric Coated) Articles-General General Drug Release Standard", Method B (100 Upm, 37 °C), jedoch mit Puffer pH 6,8 nach Europäischem Arzneibuch.

Die sigmoide Wirkstofffreisetzungscharakteristik ist dem Fachmann hinlänglich bekannt z. B. aus EP-A 0 463 877, EP 1 117 387 B1 und EP-A 0 436 370.

### Arzneiformen

Die erfindungsgemäße Zubereitung eignet sich in bekannter Weise zur Herstellung von Arzneiformen. Die Zubereitung kann z. B. in Pelletform erhalten werden, die mittels pharmazeutisch üblicher Hilfsstoffe und in an sich bekannter Weise zu multipartikulären Arzneiformen, insbesondere zu pellethaltigen Tabletten, Minitabletten, Kapseln, Sachets oder Trockensäften verarbeitet werden können.

Die Zubereitung kann bevorzugt in Form von Pellets z. B. zu einer Tablette verpresst sein.

Die Zubereitung kann z. B. insbesondere auch in Form von Pellets oder Minitabletten vorliegen, die in eine Gelatinekapsel eingefüllt sind bzw. von dieser umhüllt sind.

### BEISPIELE

### Verwendete Copolymere

### Copolymer 1:

65 Gew.-% Methylmethacrylat, 30 Gew.-% Ethylacrylat und 5 Gew.-% 2-Trimethylammoniumethlymethacrylat-Chlorid (EUDRAGIT® RS).

### Copolymer 2:

60 Gew.-% Methylmethacrylat, 30 Gew.-% Ethylacrylat und 10 Gew.-% 2-Trimethylammoniumethlymethacrylat-Chlorid (EUDRAGIT® RL).

Die Angaben in der Tabelle 1 beziehen sich auf die Trockensubstanz

### Wasserlöslickeit der Wirkstoffe:

Wasserlöslichkeit in Anlehnung nach Pharmeuropa - Technical Guide for the Elaboration of Monographs, 3rd Edition (1999), Chapter IV, Appendix IV, unter Schütteln 15 min, jedoch bei 20°C.
Theophyllin: Wasserlöslichkeit = 8,4 g/l bei 20 °C
Phenylephrin Hydrochlorid: Wasserlöslichkeit = 500 g/l bei 20 °C
Terbutalin Sulfat: Wasserlöslichkeit = 500 g/l bei 20 °C

**Tabelle 1**

| Beispiel | **1** | **2** | **3** | *4* | *5* | *6* | *7* | *8* | *9* | **10** |
|---|---|---|---|---|---|---|---|---|---|---|
| **Kerne** | | | | | | | | | | |
| Nonpareilles (600 microns) | **18,13** | **17,12** | **17,12** | *16,66* | *18,97* | *19,55* | *19,05* | *19,22* | *19.05* | **19.55** |
| Na-Succinat (anhydrous) | **23,53** | **22,22** | **22,22** | *21,62* | *24,62* | | | | *21.86* | **22,73** |
| Succinat | | | | | | *23,46* | *22,86* | *23,06* | | |
| Povidone (PVP K 30) | **2,70** | **2,55** | **2,55** | *2,48* | *2,82* | *2,74* | *2,67* | *2,69* | *2,67* | **2,64** |
| Phenylephrin HCl | **13,89** | **13,11** | **13,11** | *12,76* | *14,53* | | | | | |
| Theophyllin | | | | | | *11,73* | *11,43* | *11,53* | | |
| Terbutalin Sulfat | | | | | | | | | *12,43* | **10.46** |
| Aerosil 200* | **0,58** | **0,54** | **0,54** | *0,53* | *0,6* | *0,20* | *0,19* | *0,19* | *0,09* | **0.30** |
| Farbstoff | | | | | | *0,19* | *0,18* | *0,18* | *0,28* | **0.09** |
| Binder | | | | | | *0, 27* | *0, 27* | *0, 26* | *0.27* | **0.37** |
| **Überzüge** | | | | | | | | | | |
| EUDRAGIT^{®} RS | **26,47** | ***25,00*** | **25,00** | *24,33* | *27,69* | *23,54* | *22,95* | *34,29* | *22,95* | **23.54** |
| EUDRAGIT^{®} RL | **2,94** | **2,78** | **2,78** | *2,70* | *3,08* | *2,62* | *2.55* | *3,43* | *2,55* | **2,62** |
| SiO₂-244 FP** | **5,88** | **11,11** | | | | | | | | |
| SiO₂-160PQ*** | | | **11,11** | | | *10,47* | | | | **10,47** |
| Talkum | | | | *13, 51* | | | *12, 75* | | *12.75* | |
| GMS | | | | | *1,54* | | | *1,71* | | |
| Triethyl citrate | | **5,56** | **5,56** | *5,41* | *6,15* | *5,23* | *5,10* | *6,86* | *5,50* | **5,23** |

| | | | | | | | | | | |
|---|---|---|---|---|---|---|---|---|---|---|
| Beispiele 1 - 3, 10 = erfindungsgemäß; Beispiele 4 bis 9 = Vergleichsbeispiele Alle Angaben in Gew.-% * = Colloidales Silica, pharmazeutische Qualität, mittlere Teilchengröße ca. 12 nm ** = SiO₂- 244FP = Syloid® 244 FP = gefälltes Silica, pharmazeutische Qualität, mittlere Teilchengröße ca. 3 µm *** = SiO₂-160 PQ = Sipernat®160PQ (Degussa AG) = gefälltes Silica, pharmazeutische Qualität, mittlere Teilchengröße ca. 11 µm | | | | | | | | | | |

**Tabelle 2: Wirkstofffreisetzung in gepuffertem Medium nach USP in [%]**

| Tabelle 2: Wirkstofffreisetzung in gepuffertem Medium nach USP in [%] | | | | | | | | | | |
|---|---|---|---|---|---|---|---|---|---|---|
| Beispiel Zeit in h | **1** | **2** | **3** | *4* | *5* | *6* | *7* | *8* | *9* | **10** |
| 0 | **0.0** | **0.0** | **0.0** | *0.0* | *0.0* | *0.0* | *0.0* | *0.0* | *0,0* | **0,0** |
| 0,5 | | | | | | *1,0* | *1.1* | *1.1* | | |
| 1 | **0.0** | **0.0** | **0.0** | *0.0* | | *1.6* | *1.8* | *1.8* | | |
| 2 | **0.0** | **0.5** | **0.0** | *0.0* | *0.2* | *3.0* | *3.1* | *2.5* | *0,0* | **0,0** |
| 3 | **0.0** | **0.5** | **0.5** | | *0.9* | | *9,7* | | *0,2* | **0,5** |
| 3,5 | **3.6** | **2.2** | **7.2** | | *3.3* | | | | *0.8* | **7,2** |
| 4 | **7.3** | **12,0** | **28.1** | | *6.3* | *10.1* | *17.4* | *11.7* | *14,3* | **32,3** |
| 4,5 | **11,2** | | **52.2** | | *8,9* | | | | *25,1* | **58,1** |
| 5 | **17.0** | **60.2** | **68.5** | *3.8* | *14,0* | | | | *42,9* | **72,7** |
| 5,5 | | | *79.2* | | | | | | *55,7* | **83,0** |
| 6 | **52.7** | **87.3** | **84.1** | *11.7* | *39.0* | *37.9* | *51.8* | *42.8* | **65,1** | **89,1** |
| 7 | **78.6** | **93.6** | **91.2** | *34.9* | *68.2* | | | | *82,3* | **95,8** |
| 8 | **89.7** | **96,0** | **94.3** | *61.6* | *85.7* | *64.8* | *81.2* | *71.7* | *89,1* | |
| 9 | **94.9** | **98.2** | **96.3** | *78.8* | *93,5* | | | | *92,8* | |
| 10 | | | | *89.6* | | *83.8* | *94.4* | *84.3* | | |
| 11 | | | | *95,0* | | | | | | |
| 12 | | | | *97.8* | | *91.8* | *95.6* | *93.7* | | |
| Lag [h]* | 4,0 | 4,0 | 3,5 | *6,0* | *4,5* | *4,0* | *3,5* | *4,0* | *4,0* | 3,5 |
| Pulse [h]** | **3,5** | **2,5** | **3,5** | *4,0* | *4,5* | *8,0* | *6,5* | *7,0* | *4,5* | **2,5** |

| | | | | | | | | | | |
|---|---|---|---|---|---|---|---|---|---|---|
| Beispiele 1 - 3, 10 = erfindungsgemäß; Beispiele 4 bis 9 = Vergleichsbeispiele *= Lag [h]: Gibt die Zeit der Lag-Phase in Stunden an, in der bis 10 % des Wirkstoffs freigesetzt werden. **= Pulse [h]: Gibt die Zeit der Pulse-Phase in Stunden an, in der etwa weitere 80 % des Wirkstoffs freigesetzt werden. In den erfindungsgemäßen Beispielen 1, 2, 3 und 10 ist die Pulse-Phase auf weniger als 4 Stunden verkürzt. | | | | | | | | | | |

## Patentansprüche

1. Pharmazeutische Zubereitung, enthaltend
a) einen Kern mit einem Wirkstoff und einer organischen Säure und/oder dem Salz einer organischen Säure
b) einen den Kern umschließenden Überzug, der einen Polymeranteil aus (Meth)acrylatcopolymeren enthält, die nicht mehr als 15 Gew.-% an kationischen oder anionischen Gruppen aufweisen und der zu mindestens 60 Gew.-% eines (Meth)acrylat-Copolymeren enthält, das sich aus radikalisch polymerisierten Einheiten von 93 bis 98 Gew.-% C₁- bis C₄-Alkylestern der Acryl- oder der Methacrylsäure und 7 bis 2 Gew.-% (Meth)acrylat-Monomeren mit einer quaternären Ammoniumgruppe im Alkylrest zusammensetzt,
**dadurch gekennzeichnet, dass**
der Wirkstoff eine Wasserlöslichkeit von mindestens 10 g/l bei 20 °C aufweist und
der Überzug Siliciumdioxid-Teilchen mit einer mittleren Teilchengröße im Bereich von 1 bis 50 µm enthält.

2. Zubereitung nach Anspruch 1, **dadurch gekennzeichnet, dass** der Polymeranteil des Überzugs eine Mischung ist aus
60 bis 99 Gew.-% eines (Meth)acrylat-Copolymeren, das zu 93 bis 98 Gew.-% aus C₁- bis C₄-Alkylestern der Acryl- oder der Methacrylsäure und 7 bis 2 Gew.-% (Meth)acrylat-Monomeren mit einer quaternären Ammoniumgruppe im Alkylrest aufgebaut ist, und
1 - 40 Gew.-% eines (Meth)acrylat-Copolymeren, das zu 85 bis weniger als 93 Gew.-% aus C₁- bis C₄-Alkylestern der Acryl- oder der Methacrylsäure und mehr als 7 bis 15 Gew.-% (Meth)acrylat-Monomeren mit einer quaternären Ammoniumgruppe im Alkylrest aufgebaut ist.

3. Zubereitung nach Anspruch 1, **dadurch gekennzeichnet, dass** der der Polymeranteil des Überzugs b) eine Mischung ist aus
60 bis 99 Gew.-% eines (Meth)acrylat-Copolymeren, das zu 93 bis 98 Gew.-% aus C₁- bis C₄-Alkylestern der Acryl- oder der Methacrylsäure und 7 bis 2 Gew.-% (Meth)acrylat Monomeren mit einer quaternären Ammoniumgruppe im Alkylrest besteht, und
1 - 40 Gew.-% eines (Meth)acrylat-Copolymeren, das zu 95 bis 100 Gew.-% aus C₁- bis C₄-Alkylestern der Acryl- oder der Methacrylsäure und 0 - 5 Gew.-% aus Acryl- oder Methacrylsäure aufgebaut ist.

4. Zubereitung nach einem oder mehreren der Ansprüche 1 bis 3, **dadurch gekennzeichnet, dass** als (Meth)acrylat-Monomer mit einer quaternären Ammoniumgruppe im Alkylrest Trimethylammoniumethylmethacrylat-Chlorid im(Meth)acrylatcopolymeren enthalten ist.

5. Zubereitung nach einem oder mehreren der Ansprüche 1 bis 4, **dadurch gekennzeichnet, dass** der Kern und/oder der Überzug weitere pharmazeutisch übliche Hilfsstoffe enthält bzw. enthalten.

6. Zubereitung nach einem oder mehreren der Ansprüche 1 bis 5, **dadurch gekennzeichnet, dass** der Polymeranteil des Überzugs bezogen auf das Gewicht des Kerns 10 bis 200 Gew.-% beträgt.

7. Zubereitung nach einem oder mehreren der Ansprüche 1 bis 5, **dadurch gekennzeichnet, dass** der Polymeranteil des Überzugs zusammen mit dem Anteil des enthaltenen SiO₂ bezogen auf den Überzug insgesamt 10 bis 100 Gew.-% ausmacht.

8. Zubereitung nach einem oder mehreren der Ansprüche 1 bis 7, **dadurch gekennzeichnet, dass** als organische Säure und/oder als Salz einer organischen Säure Citronensäure, Fumarsäure, Ameisensäure, Essigsäure, Maleinsäure, Bernsteinsäure, Weinsäure, Glutarsäure oder Milchsäure oder deren Ammonium-, Lithium-, Natrium- oder Kalium-Salze oder Mischungen der genannten Substanzen eingesetzt werden.

9. Zubereitung nach einem oder mehreren der Ansprüche 1 bis 8, **dadurch gekennzeichnet, dass** es sich bei den Siliciumdioxid-Teilchen um gefälltes Siliciumdioxid handelt.

10. Zubereitung nach einem oder mehreren der Ansprüche 1 bis 9, **dadurch gekennzeichnet, dass** 5 bis 50 Gew.-% Siliciumdioxid-Teilchen bezogen auf das oder die im Überzug enthaltenen (Meth)acrylatcopolymere enthalten sind.

11. Zubereitung nach einem oder mehreren der Ansprüche 1 bis 10, **dadurch gekennzeichnet, dass** sie zu einer Tablette verpresst ist.

12. Zubereitung nach einem oder mehreren der Ansprüche 1 bis 10, **dadurch gekennzeichnet, dass** sie von einer Gelatinekapsel umhüllt ist.

13. Zubereitung nach einem oder mehreren der Ansprüche 1 bis 12, **dadurch gekennzeichnet, dass** die organische Säure und/oder das Salz der organischen Säure die äußere Schicht des Kerns bildet.

14. Zubereitung nach einem oder mehreren der Ansprüche 1 bis 13, **dadurch gekennzeichnet, dass** sie zusätzlich mit einem (Meth)acrylat-Copolymer, welches 10 - 60 Gew.-% Methacrylsäurereste enthält, umhüllt ist.

15. Zubereitung nach einem oder mehreren der Ansprüche 1 bis 14, **dadurch gekennzeichnet, dass** einer oder mehrere der folgenden Wirkstoffe gegebenenfalls in der Form der wasserlöslichen, pharmazeutisch eingesetzten Salze enthalten sind: Acebutolol, Amitryptylin, Aripiprazol, Atenolol, Atropin, Betaxolol, Bisoprolol, Bupavacaine, Buproprion, Butabarbital, Carteolol, Carvedilol, Cefazoline, Cefotaxime, Chlorphenaramine, Chlorpromazine, Clindamycin, Codein, Diltiazem, Dimercaprol, Diphenhydarmine, Dopamine, Doxylamine, Duloxetine, Flexainid, Fluoxetin, Fluphenazine, Flurazepam, Gentamycin, Hydralazine, Hydrocortisone, Hydroquinone, Hyoscyamine, Isoniazid, Isoproterenol, Kanamycin, Labetolol, Lisinopril, Metipranolol, Mexiletin, Morphine, Nadolol, Neomycin, Norepinephrine, Nortryptylin, Ondansetron, Oxprenolol, Oxymetazoline, Oxymorphone, Paroxetin, Penbutolol, Phenylephrin, Pindolol, Prednisolone, Primaquine, Propranolol, Pyrrocaine, Sotalol, Sulphadiazine, Tamoxifen, Terbutalin, Timolol, Tramadol, Trazodon, Triflupromazine, Tetracycline, Tubocurarin, Venlafaxin und/oder Verapamil.

16. Verwendung einer pharmazeutischen Zubereitung nach einem oder mehreren der Ansprüche 1 bis 15 zur Herstellung einer Arzneiform für Wirkstoffe, die eine Wasserlöslichkeit von mindestens 10 g/l bei 20 °C aufweisen, wobei die Arzneiform eine sigmoide Wirkstofffreisetzungscharakteristik mit einer Lag-Phase, einer Pulse-Phase und einer Auslaufphase aufweist, **gekennzeichnet durch** eine Wirkstofffreisetzung, im Paddlegerät mit 100 Upm in Puffer pH 6,8 nach Europäischem Arzneibuch, während der Lag-Phase annähernd 10 % und einer anschließenden Wirkstofffreisetzung in der Pulse-Phase von annähernd weiteren 80 % innerhalb von weniger als 4 Stunden.

## Claims

1. Pharmaceutical preparation comprising
a) a core with an active ingredient and with an organic acid and/or the salt of an organic acid
b) a coating which envelops the core and which comprises a polymer content of (meth)acrylate copolymers which have not more than 15% by weight of cationic or anionic groups, and which comprises at least 60% by weight of a (meth)acrylate copolymer which is composed of free-radically polymerized units of 93 to 98% by weight C₁- to C₄-alkyl esters of acrylic or of methacrylic acid and 7 to 2% by weight (meth)acrylate monomers having a quaternary ammonium group in the alkyl radical,
**characterized in that**
the active ingredient has a solubility in water of at least 10 g/l at 20°C and
the coating comprises silicon dioxide particles having an average particle size in the range from 1 to 50 µm.

2. Preparation according to Claim 1, **characterized in that** the polymer content of the coating is a mixture of
60 to 99% by weight of a (meth)acrylate copolymer which is composed of 93 to 98% by weight C₁- to C₄-alkyl esters of acrylic or of methacrylic acid and 7 to 2% by weight (meth)acrylate monomers having a quaternary ammonium group in the alkyl radical, and
1-40% by weight of a (meth)acrylate copolymer which is composed of 85 to less than 93% by weight C₁- to C₄-alkyl esters of acrylic or of methacrylic acid and more than 7 to 15% by weight (meth)acrylate monomers having a quaternary ammonium group in the alkyl radical.

3. Preparation according to Claim 1, **characterized in that** the polymer content of the coating b) is a mixture of
60 to 99% by weight of a (meth)acrylate copolymer which is composed of 93 to 98% by weight C₁- to C₄-alkyl esters of acrylic or of methacrylic acid and 7 to 2% by weight (meth)acrylate monomers having a quaternary ammonium group in the alkyl radical, and
1-40% by weight of a (meth)acrylate copolymer which is composed of 95 to 100% by weight C₁- to C₄-alkyl esters of acrylic or of methacrylic acid and 0-5% by weight acrylic or methacrylic acid.

4. Preparation according to one or more of Claims 1 to 3, **characterized in that** trimethylammoniumethyl methacrylate chloride is present as (meth)acrylate monomer having a quaternary ammonium group in the alkyl radical in the (meth)acrylate copolymer.

5. Preparation according to one or more of Claims 1 to 4, **characterized in that** the core and/or the coating comprises or comprise further pharmaceutically usual excipients.

6. Preparation according to one or more of Claims 1 to 5, **characterized in that** the polymer content of the coating amounts to 10 to 200% by weight based on the weight of the core.

7. Preparation according to one or more of Claims 1 to 5, **characterized in that** the polymer content of the coating together with the proportion of contained SiO₂ totals 10 to 100% by weight based on the coating.

8. Preparation according to one or more of Claims 1 to 7, **characterized in that** citric acid, fumaric acid, formic acid, acetic acid, maleic acid, succinic acid, tartaric acid, glutaric acid or lactic acid or their ammonium, lithium, sodium or potassium salts or mixtures of the said substances are employed as organic acid and/or as salt of an organic acid.

9. Preparation according to one or more of Claims 1 to 8, **characterized in that** the silicon dioxide particles comprise precipitated silicon dioxide.

10. Preparation according to one or more of Claims 1 to 9, **characterized in that** 5 to 50% by weight silicon dioxide particles based on the (meth)acrylate copolymer(s) present in the coating are present.

11. Preparation according to one or more of Claims 1 to 10, **characterized in that** it is compressed to give a tablet.

12. Preparation according to one or more of Claims 1 to 10, **characterized in that** it is enveloped by a gelatin capsule.

13. Preparation according to one or more of Claims 1 to 12, **characterized in that** the organic acid and/or the salt of the organic acid forms the outer layer of the core.

14. Preparation according to one or more of Claims 1 to 13, **characterized in that** it is additionally enveloped by a (meth)acrylate copolymer which comprises 10-60% by weight methacrylic acid residues.

15. Preparation according to one or more of Claims 1 to 14, **characterized in that** one or more of the following active ingredients are present, where appropriate in the form of the water-soluble, pharmaceutically employed salts: acebutolol, amitriptyline, aripiprazole, atenolol, atropine, betaxolol, bisoprolol, bupivacaine, bupropion, butabarbital, carteolol, carvedilol, cefazoline, cefotaxime, chlorpheniramine, chlorpromazine, clindamycin, codeine, diltiazem, dimercaprol, diphenhydramine, dopamine, doxylamine, duloxetine, flecainide, fluoxetine, fluphenazine, flurazepam, gentamycin, hydralazine, hydrocortisone, hydroquinone, hyoscyamine, isoniazid, isoproterenol, kanamycin, labetolol, lisinopril, metipranolol, mexiletine, morphine, nadolol, neomycin, norepinephrine, nortriptyline, ondansetron, oxprenolol, oxymetazoline, oxymorphone, paroxetine, penbutolol, phenylephrine, pindolol, prednisolone, primaquine, propranolol, pyrrocaine, sotalol, sulphadiazine, tamoxifen, terbutaline, timolol, tramadol, trazodone, triflupromazine, tetracycline, tubocurarine, venlafaxine and/or verapamil.

16. Use of a pharmaceutical preparation according to one or more of Claims 1 to 15 for producing a pharmaceutical form for active ingredients which have a solubility in water of at least 10 g/l at 20°C, with the pharmaceutical form showing sigmoidal active ingredient release characteristics with a lag phase, a pulse phase and a run-out phase, **characterized by** an active ingredient release in the paddle apparatus at 100 rpm in buffer of pH 6.8 according to the European Pharmacopoeia of approximately 10% during the lag phase and a subsequent active ingredient release of approximately a further 80% within less than 4 hours in the pulse phase.

## Revendications

1. Composition pharmaceutique, contenant
a) un noyau comprenant une substance active et un acide organique et/ou le sel d'un acide organique
b) un revêtement entourant le noyau, qui contient une proportion polymère constituée par des copolymères de (méth)acrylate qui ne présentent pas plus de 15% en poids de groupes cationiques ou anioniques et qui contient à raison d'au moins 60% en poids un copolymère de (méth)acrylate, composé d'unités polymérisées par voie radicalaire de 93 à 98% en poids d'esters alkyliques en C₁-C₄ de l'acide acrylique ou méthacrylique et de 7 à 2% en poids de monomères de (méth)acrylate présentant un groupe d'ammonium quaternaire dans le radical alkyle,
**caractérisée en ce que** la substance active présente une solubilité dans l'eau d'au moins 10 g/l à 20°C et le revêtement contient des particules de dioxyde de silicium présentant une grosseur moyenne des particules dans la plage de 1 à 50 µm.

2. Composition selon la revendication 1, **caractérisée en ce que** la proportion polymère du revêtement est un mélange
de 60 à 99% en poids d'un copolymère de (méth)acrylate qui est formé par 93 à 98% en poids d'esters alkyliques en C₁-C₄ de l'acide acrylique ou méthacrylique et par 7 à 2% en poids de monomères de (méth)acrylate présentant un groupe d'ammonium quaternaire dans le radical alkyle, et
de 1-40% en poids d'un copolymère de (méth)acrylate qui est formé par 85 à moins de 93% en poids d'esters alkyliques en C₁-C₄ de l'acide acrylique ou méthacrylique et par plus de 7 à 15% en poids de monomères de (méth)acrylate présentant un groupe d'ammonium quaternaire dans le radical alkyle.

3. Composition selon la revendication 1, **caractérisée en ce que** la proportion polymère du revêtement b) est un mélange
de 60 à 99% en poids d'un copolymère de (méth)acrylate qui est formé par 93 à 98% en poids d'esters alkyliques en C₁-C₄ de l'acide acrylique ou méthacrylique et par 7 à 2% en poids de monomères de (méth)acrylate présentant un groupe d'ammonium quaternaire dans le radical alkyle, et
de 1-40% en poids d'un copolymère de (méth)acrylate qui est formé par 95 à 100% en poids d'esters alkyliques en C₁-C₄ de l'acide acrylique ou méthacrylique et par 0 à 5% en poids d'acide acrylique ou méthacrylique.

4. Composition selon l'une ou plusieurs des revendications 1 à 3, **caractérisée en ce qu'**elle contient comme monomère de (méth)acrylate présentant un groupe d'ammonium quaternaire dans le radical alkyle du chlorure de méthacrylate de triméthylammoniuméthyle dans des copolymères de (méth)acrylate.

5. Composition selon l'une ou plusieurs des revendications 1 à 4, **caractérisée en ce que** le noyau et/ou le revêtement contien(nen)t d'autres adjuvants pharmaceutiquement usuels.

6. Composition selon l'une ou plusieurs des revendications 1 à 5, **caractérisée en ce que** la proportion polymère du revêtement est de 10 à 200% en poids par rapport au poids du noyau.

7. Composition selon l'une ou plusieurs des revendications 1 à 5, **caractérisée en ce que** la proportion polymère du revêtement, ensemble avec la proportion du SiO₂ contenu, par rapport au revêtement, représente au total 10 à 100% en poids.

8. Composition selon l'une ou plusieurs des revendications 1 à 7, **caractérisée en ce qu'**on utilise comme acide organique et/ou comme sel d'un acide organique l'acide citrique, l'acide fumarique, l'acide formique, l'acide acétique, l'acide maléique, l'acide succinique, l'acide tartrique, l'acide glutarique ou l'acide lactique ou leurs sels d'ammonium, de lithium, de sodium ou de potassium ou des mélanges des substances mentionnées.

9. Composition selon l'une ou plusieurs des revendications 1 à 8, **caractérisée en ce qu'**il s'agit, pour les particules de dioxyde de silicium, de dioxyde de silicium précipité.

10. Composition selon l'une ou plusieurs des revendications 1 à 9, **caractérisée en ce qu'**elle contient 5 à 50% en poids de particules de dioxyde de silicium par rapport au(x) copolymère(s) de (méth)acrylate contenu(s) dans le revêtement.

11. Composition selon l'une ou plusieurs des revendications 1 à 10, **caractérisée en ce qu'**elle est pressée en un comprimé.

12. Composition selon l'une ou plusieurs des revendications 1 à 10, **caractérisée en ce qu'**elle est enveloppée par une capsule de gélatine.

13. Composition selon l'une ou plusieurs des revendications 1 à 12, **caractérisée en ce que** l'acide organique et/ou le sel de l'acide organique forme la couche extérieure du noyau.

14. Composition selon l'une ou plusieurs des revendications 1 à 13, **caractérisée en ce qu'**elle est en outre enveloppée par un copolymère de (méth)acrylate qui contient 10-60% en poids de radicaux d'acide méthacrylique.

15. Composition selon l'une ou plusieurs des revendications 1 à 14, **caractérisée en ce qu'**une ou plusieurs des substances actives suivantes sont contenues, le cas échéant sous la forme des sels solubles dans l'eau, utilisés pharmaceutiquement : acébutolol, amitriptyline, aripiprazole, aténolol, atropine, bétaxolol, bisoprolol, bupivacaïne, bupropion, butabarbital, cartéolol, carvedilol, céfazoline, céfotaxime, chlorpheniramin, chlorpromazine, clindamycine, codéine, diltiazem, dimercaprol, diphénhydramine, dopamine, doxylamine, duloxétine, flécainide, fluoxétine, fluphénazine, flurazepam, gentamycine, hydralazine, hydrocortisone, hydroquinone, hyoscyamine, isoniazide, isoproterenol, kanamycine, labétolol, lisinopril, métipranolol, mexiletine, morphine, nadolol, néomycine, norépinéphrine, nortriptyline, ondansétron, oxprenolol, oxymétazoline, oxymorphone, paroxétine, penbutolol, phényléphrine, pindolol, prednisolone, primaquine, propranolol, pyrrocaine, sotalol, sulfadiazine, tamoxifène, terbutaline, timolol, tramadol, trazodone, triflupromazine, tétracycline, tubocurarine, venlafaxine et/ou vérapamil.

16. Utilisation d'une composition pharmaceutique selon l'une ou plusieurs des revendications 1 à 15 pour la préparation d'une forme médicamenteuse pour des substances actives qui présentent une solubilité dans l'eau d'au moins 10 g/l à 20°C, la forme médicamenteuse présentant une caractéristique sigmoïdale de libération de la substance active avec une phase de retard, une phase d'impulsion et une phase de fin, **caractérisée par** une libération de la substance active dans un appareil à palette à 100 t/min dans un tampon de pH 6,8 selon la pharmacopée européenne, pendant la phase de retard d'approximativement 10% et par une libération consécutive de la substance active dans la phase d'impulsion d'approximativement 80% en moins de 4 heures.
